(19) Europäisches Patentamt
European Patent Office
Office européen des brevets

(11) Veröffentlichungsnummer: **0 111 700**
**B1**

(12) # EUROPÄISCHE PATENTSCHRIFT

(45) Veröffentlichungstag der Patentschrift: **06.08.86**

(51) Int. Cl.⁴: **C 01 B 33/28**, B 01 J 29/28, C 07 C 1/20, C 07 C 11/02

(21) Anmeldenummer: **83110895.6**

(22) Anmeldetag: **02.11.83**

(54) **Titan-, zirkon- und/oder hafniumhaltige Zeolithe und Verfahren zu ihrer Herstellung sowie ihre Verwendung.**

(30) Priorität: **05.11.82 DE 3240869**

(43) Veröffentlichungstag der Anmeldung: **27.06.84 Patentblatt 84/26**

(45) Bekanntmachung des Hinweises auf die Patenterteilung: **06.08.86 Patentblatt 86/32**

(84) Benannte Vertragsstaaten: **BE DE FR GB IT NL**

(56) Entgegenhaltungen:
**DE - A - 2 924 870**
**DE - A - 3 141 283**
**DE - A - 3 141 285**
**DE - A - 3 217 322**
**DE - A - 3 217 323**

(73) Patentinhaber: **HOECHST AKTIENGESELLSCHAFT, Postfach 80 03 20, D-6230 Frankfurt am Main 80 (DE)**

(72) Erfinder: **Baltes, Herbert, Dr., Johannesallee 24, D-6230 Frankfurt am Main 80 (DE)**
Erfinder: **Litterer, Heinz, Dr., Albert-Schweitzer-Allee 39, D-6200 Wiesbaden (DE)**
Erfinder: **Leupold, Ernst Ingo, Dr., Am Zäunefeld 15, D-6392 Neu-Anspach (DE)**
Erfinder: **Wunder, Friedrich, Dr., Jahnstrasse 46, D-6093 Flörsheim am Main (DE)**
Erfinder: **Ebertz, Wolfgang, Dr., Nelkenweg 12, D-5047 Wesseling (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Als Zeolithe bezeichnet man vor allem kristalline Aluminosilicate, bei denen durch eine dreidimensionale Verknüpfung von $SiO_4$- und $AlO_4$-Tetraedern regelmässige Strukturen mit Hohlräumen und Poren entstehen. Im hydratisierten Zustand sind diese Poren und Hohlräume mit Wasser gefüllt. Dieses lässt sich ohne Beeinflussung der Kristallstruktur entfernen oder durch andere Moleküle ersetzen. Die negativen Ladungen der $AlO_4$-Tetraeder werden durch Kationen kompensiert. Diese können, wenn gewünscht, gegen andere Kationen ausgetauscht werden. Die geschilderten Eigenschaften ermöglichen die Verwendung der Zeolithe als Ionenaustauscher, Adsorbentien und Katalysatoren (D.W. Breck: Zeolite Molecular Sieves, 1974).

Zeolithe des X-, Y, Mordenit-, Erionit- und Offretit-Typs beispielsweise besitzen als Katalysatoren für Umwandlungsreaktionen von Kohlenwasserstoffen wie Cracken, Hydrocracken oder Isomerisierungen beträchtliches technisches Interesse. Zeolithe vom Pentasil-Typ (z.B. Zeolith ZSM-5) gewinnen als Katalysatoren für die Umwandlung von Methanol zu Kohlenwasserstoffen steigende Bedeutung.

Aufgrund der zahlreichen Einsatzmöglichkeiten als Katalysatoren besteht grosses Interesse an neuen Zeolithen mit spezifischen katalytischen Eigenschaften. Beispielsweise erhält man sehr interessante Zeolithe, wenn man anstelle von Aluminium und/oder Silicium andere Elemente in das Zeolith-Gerüst einbaut. So wurden unter anderem Zeolithe der Pentasil-Reihe bekannt, die Bor (DE-A-2 830 787), Eisen (DE-A-2 831 611), Arsen (DE-A-2 830 830), Antimon (DE-A-2 830 787), Vanadin (DE-A-2 831 631), Chrom (DE-A-2 831 630) oder Gallium (BE-A-882 484) auf Tetraederplätzen enthalten. Auch wurden Titanosilicate (US-A-3 329 481 und DE-A-3 047 798) und Zirkonosilicate (US-A-3 329 480) mit Zeolithstruktur bekannt.

Weiterhin wurden bereits beschrieben: borhaltige Zeolithe, gallium- und/oder indiumhaltige Zeolithe, titanhaltige Zeolithe sowie zirkon- und/oder hafniumhaltige Zeolithe (deutsche Offenlegungsschriften 3 134 316, 3 134 317, 3 136 686, 3 136 684, 3 141 283, 3 141 285, 3 217 324, 3 217 323).

Gegenstand der Erfindung sind titan-, zirkon- und/oder hafniumhaltige Zeolithe, die dadurch gekennzeichnet sind, dass sie

a) Silicium, Aluminium, Natrium, Kalium, eine organische Ammoniumverbindung und mindestens ein Element aus der Gruppe Titan, Zirkon und Hafnium in folgendem Verhältnis enthalten

$$(SiO_2 + MO_2) : (0{,}02 — 0{,}30)\ Al_2O_3 : (0{,}05 — 0{,}30)\ (Na_2O + K_2O) : (0{,}01 — 0{,}30)\ R_2O,$$

ausgedrückt in Molverhältnissen von Oxiden, wobei M gleich Titan, Zirkon und/oder Hafnium ist und R Ammoniumreste der allgemeinen Formeln $(HOCH_2CH_2)_4N$, $(HOCH_2CH_2)_3R^1N$ oder $(HOCH_2CH_2)_2R^1R^2N$ bezeichnet und die Reste $R^1$ und $R^2$ gleich oder verschieden sein können und Alkyl, substituiertes Alkyl, Cycloalkyl, substituiertes Cycloalkyl, Aryl, substituiertes Aryl oder Wasserstoff bedeuten und

b) im Röntgenbeugungsdiagramm die in Tabelle 1 aufgeführten charakteristischen Signale aufweisen:

TABELLE 1

| Netzebenenabstände d (Å) | relative Intensität $I/I_o$ |
|---|---|
| 11,5 ± 0,3 | stark bis sehr stark |
| 9,2 ± 0,2 | schwach |
| 7,6 ± 0,2 | schwach bis mittel |
| 6,6 ± 0,1 | mittel bis stark |
| 6,3 ± 0,1 | schwach |
| 5,7 ± 0,1 | schwach |
| 5,35 ± 0,1 | schwach |
| 4,56 ± 0,1 | schwach bis mittel |
| 4,32 ± 0,1 | stark |
| 4,16 ± 0,1 | schwach |
| 3,81 ± 0,1 | mittel bis stark |
| 3,75 ± 0,1 | stark bis sehr stark |
| 3,59 ± 0,1 | stark bis sehr stark |
| 3,30 ± 0,1 | mittel |
| 3,15 ± 0,1 | mittel |
| 2,86 ± 0,1 | stark bis sehr stark |
| 2,80 ± 0,1 | schwach bis mittel |
| 2,67 ± 0,1 | schwach bis mittel |
| 2,49 ± 0,1 | schwach bis mittel |

Hierbei bedeutet $I_o$ die Intensität des stärksten Signals, und es gilt $\frac{SiO_2}{SiO_2 + MO_2} = 0{,}4 — 0{,}99$, ausgedrückt in Molverhältnissen der Oxide. Für die Angabe der Intensitäten in Tabelle 1 gilt:

| relative Intensität | 100 $I/I_o$ |
|---|---|
| sehr stark | 80 - 100 |
| stark | 50 - 80 |
| mittel | 20 - 50 |
| schwach | 0 - 20 |

Vorzugsweise besitzen die erfindungsgemässen Zeolithe die folgende Zusammensetzung, ausgedrückt in Molverhältnissen der Oxide:

$$(SiO_2 + MO_2) : (0{,}08 — 0{,}18)\ Al_2O_3 : (0{,}05 — 030)\ (Na_2O + K_2O) : (0{,}01 — 0{,}30)\ R_2O,$$

wobei M gleich Titan, Zirkon und/oder Hafnium ist.

Dabei hat R die oben angegebene Bedeutung, vorzugsweise ist $R = (HOCH_2CH_2)_3R^1N$.

$R^1$ und $R^2$ haben die oben angegebene Bedeutung, vorzugsweise sind sie Alkylreste mit maximal jeweils fünf C-Atomen oder Wasserstoff, insbesondere Methyl, Ethyl, oder Wasserstoff. $R^1$ und $R^2$ können verschieden sein, vorzugsweise gilt jedoch $R^1 = R^2$, insbesondere $R^1 = R^2 =$ Methyl.

Vorzugsweise gilt für das Verhältnis von Silicium zu Titan, Zirkon und/oder Hafnium in den erfindungsgemässen Zeolithen:

$$\frac{SiO_2}{SiO_2 + MO_2} = 0{,}7 — 0{,}99,$$

ausgedrückt in Molverhältnissen der Oxide, wobei M gleich Titan, Zirkon und/oder Hafnium ist.

Die erfindungsgemässen neuen Zeolithe besitzen eine dem Erionit (L.W. Staples, J.A. Gard, Mineralogical Magazine, Band 32, Jahrgang 1959, Seite 261 f), bzw. den synthetischen Zeolithen T (US-A-2 950 952) und ZSM-34 (DE-A-2 749 024) ähnliche Struktur, unterscheiden sich jedoch von diesen in der Zusammensetzung, insbesondere durch den Gehalt an mindestens einem Element aus der Gruppe Titan, Zirkon und Hafnium sowie durch die Art der organischen Ammoniumverbindung.

Ein weiteres kristallines Aluminosilicat dieses Strukturtyps wurde kürzlich in der deutschen Offenlegungsschrift 3 217 322 beschrieben. Von diesem Aluminosilikat unterscheiden sich die erfindungsgemässen Zeolithe durch den Gehalt an Titan, Zirkon und/oder Hafnium sowie durch unterschiedliche katalytische Eigenschaften.

Von den Titanosilicaten gemäss US-A-3 329 481 und DE-A-3 047 798, den Zirkonosilicaten gemäss US-A-3 329 480, den titanhaltigen Zeolithen gemäss der deutschen Offenlegungsschrift 3 141 283 sowie den zirkon- und/oder hafniumhaltigen Zeolithen gemäss der deutschen Offenlegungsschrift 3 141 285 unterscheiden sich die erfindungsgemässen titan-, zirkon- und/oder hafniumhaltigen Zeolithe durch die Struktur sowie durch die Art der organischen Ammoniumverbindung.

Von den titanhaltigen bzw. zirkon- und/oder hafniumhaltigen Zeolithen mit ähnlicher Struktur (deutsche Offenlegungsschrift 3 136 686 bzw. 3 136 684) unterscheiden sich die erfindungsgemässen titan-, zirkon- und/oder hafniumhaltigen Zeolithe durch die Art der organischen Ammoniumverbindung. Die erfindungsgemässen Zeolithe zeichnen sich ferner durch eine unterschiedliche Kristallform sowie durch wesentlich grössere Kristallite aus.

Die erfindungsgemässen Zeolithe lassen sich herstellen, indem man eine Ammoniumverbindung RX mit Aluminium-, Silicium-, Natrium-, Kaliumverbindungen und Wasser sowie mindestens einer Verbindung aus der Gruppe der Titan-, Zirkon-, und/oder Hafniumverbindungen mischt und in einem geschlossenen Gefäss erhitzt. Dabei hat R die oben angegebene Bedeutung. Dem Gemisch können darüberhinaus vor dem Erhitzen Impfkristalle zugesetzt werden.

Die Ausgangsverbindungen werden im allgemeinen in folgendem Verhältnis eingesetzt, ausgedrückt in Molverhältnissen der Oxide:

$(SiO_2 + MO_2) : (0,02 — 0,30)\ Al_2O_3 : (0,02 — 0,70)\ Na_2O : (0,02 — 0,30)\ K_2O : (0,02 — 0,5)\ R_2O : (10 — 90)\ H_2O$,

vorzugsweise im Verhältnis

$(SiO_2 + MO_2) : (0,02 — 0,18)\ Al_2O_3 : (0,10 — 0,60)\ Na_2O : (0,04 — 0,20)\ K_2O : (0,10 — 0,40)\ R_2O : (10 — 40)\ H_2O$,

wobei M gleich Titan, Zirkon und/oder Hafnium ist und R die oben angegebene Bedeutung hat.

Dabei gilt:

$$\frac{SiO_2}{SiO_2 + MO_2} = 0,4 — 0,99,$$

vorzugsweise

$$\frac{SiO_2}{SiO_2 + MO_2} = 0,6 — 0,99,$$

ausgedrückt in Molverhältnissen der Oxide, wobei M gleich Titan, Zirkon und/oder Hafnium ist.

Als Ammoniumverbindung RX können alle wasserlöslichen Salze von R eingesetzt werden. X kann beispielsweise bedeuten: Hydroxyl, Chlorid, Bromid, Iodid, Sulfat, Phosphat, Sulfonat, Carboxylat, Carbonat und Sulfit.

Die Ammoniumverbindung RX kann als Substanz eingesetzt werden. Vorzugsweise wird sie jedoch in situ im Reaktionsgemisch erzeugt, indem man ein Gemisch aus Triethanolamin und/oder Diethanolamin einerseits und einer Verbindung der allgemeinen Formel $R^1Y$ andererseits einsetzt, wobei $R^1$ die oben angegebene Bedeutung hat. Y ist im allgemeinen Hydroxyl, Monoalkylsulfat, Halogenid oder Sulfonat, insbesondere Hydroxyl.

$R^1Y$ ist vorzugsweise Methanol, Ethanol, Propanol, Butanol, Ethylenglykol, 1,2-Propylenglykol, Dimethylsulfat, Diethylsulfat, Methyliodid, Ethyliodid, Propyliodid, p-Toluolsulfonsäuremethylester, p-Toluolsulfonsäureethylester oder p-Toluolsulfonsäurepropylester. Insbesondere ist $R^1Y$ Methanol, Ethanol oder Ethylenglykol. Das Molverhältnis $R^1Y$ zu Amin (Triethanolamin und/oder Diethanolamin) beträgt im allgemeinen 0,5 bis 20, vorzugsweise 1 bis 10, insbesondere 4 bis 10.

Die erfindungsgemässen Zeolithe lassen sich jedoch auch in Abwesenheit einer Verbindung der allgemeinen Formel $R^1Y$, d.h. nur in Gegenwart von Triethanolamin und/oder Diethanolamin mit zufriedenstellender Kristallinität herstellen.

Als Titan-, Zirkon- und Hafniumverbindungen können beispielsweise eingesetzt werden: Titanhalogenide, Titansulfat, Titanoxidsulfat, Titanalkoholate, Natriumtitanat, Kaliumtitanat, Titandioxid, Zirkonhalogenide, Zirkonsulfat, Zirkonalkoholate, Zirkonnitrat, Zirkondioxid, Zirkonylhalogenide, Zirkonylsulfat, Natriumzirkonat, Kaliumzirkonat, Hafniumhalogenide, Hafniumdioxid, Hafniumoxychlorid. Aber auch andere Titan-, Zirkon- und Hafniumverbindungen eignen sich für die Herstellung der erfindungsgemässen Zeolithe.

Als Silicium-, Aluminium-, Natrium- und Kaliumverbindungen können beispielsweise eingesetzt werden: Kieselsäuregel, Kaliumsilicat, Natriumsilicat, Natriumaluminat, Kaliumaluminat, Aluminiumhalogenide, Aluminiummetahydroxid, Kaliumhydroxid, Kaliumsulfat, Kaliumhalogenide, Natriumhydroxid, Natriumsulfat, Natriumhalogenide. Aber auch andere Silicium-, Aluminium-, Kalium- und Natriumverbindungen eignen sich für die Herstellung der erfindungsgemässen Zeolithe.

Das Gemisch der jeweils gewählten Verbindungen mit Wasser wird im allgemeinen 18 bis 1000 Stunden, vorzugsweise 24 bis 500 Stunden lang auf eine Temperatur zwischen 80 und 200°C, vorzugsweise zwischen 110 und 160°C, in einem geschlossenen Gefäss erhitzt.

Die gebideten Zeolithe werden in üblicher Weise, z.B. durch Filtration, isoliert, gewaschen und getrocknet. Sie können nach bekannten Methoden in die katalytisch aktiven Formen überführt werden, z.B. durch Kalzinierung und/oder Ionenaustausch (D.W. Breck, Zeolite Molecular Sieves, 1974).

Die erfindungsgemässen Zeolithe zeichnen sich nach ihrer Überführung in die katalytisch aktive Form insbesondere aus durch eine hohe Selektivität und durch eine geringe Koksabscheidung bei der Umwandlung von Methanol in niedere Olefine. Diese Reaktion führt man beispielsweise bei Temperaturen von 350-430°C und einem Wasseranteil im Methanol von 0 bis 80 Gew.-% oder mit Rohmethanol durch.

Die Erfindung soll durch die folgenden Beispiele erläutert werden, wobei die Beispiele aber in keiner Weise einschränkend sein sollen. Alle angegebenen Röntgenbeugungsdaten wurden mit einem computergesteuerten Pulverdiffraktometer D-500 der Firma Siemens aufgenommen. Es wurde Kupfer-K-alpha-Strahlung verwandt.

*Beispiel 1*

17,92 g Natriumaluminat (54 Gew.-% $Al_2O_3$, 41 Gew.-% $Na_2O$), 9,5 g Natriumhydroxid, 10 g Kaliumhydroxid, 77,6 g Triethanolamin und 56 g Ethylenglykol werden in 240 ml Wasser gelöst (Lösung A). 14,2 g Titanethanolat $Ti(OC_2H_5)_4$ werden in 40 g Ethylenglykol gelöst (Lösung B). In die Lösung A werden nun unter intensivem Rühren zuerst 178 g 40 gew.-%iges kolloidales Kieselgel und anschliessend die Lösung B eingebracht. Die entstandene Mischung wird homogenisiert und anschliessend in einem Rührautoklaven 120 h auf 150°C erhitzt. Das entstandene Produkt wird abfiltriert, mit Wasser gewaschen und bei 120°C getrocknet.

Das Produkt besitzt das in Tabelle 2 wiedergegebene Röntgenbeugungsmuster.

Die chemische Analyse ergibt folgende Zusammensetzung, ausgedrückt in Molverhältnissen von Oxiden:

$SiO_2 : 0,147\ Al_2O_3 : 0,058\ TiO_2 : 0,073\ Na_2O : 0,091\ K_2O : 0,060\ R_2O$,

wobei R gleich $(HOCH_2CH_2)_4N$ ist.

TABELLE 2

| Netzebenenabstände d (Å) | relative Intensität 100 $I/I_o$ |
|---|---|
| 11,3 | 84 |
| 9,2 | 4 |
| 7,55 | 17 |
| 6,60 | 62 |
| 6,29 | 6 |
| 5,70 | 4 |
| 5,34 | 3 |
| 4,51 | 12 |
| 4,28 | 59 |
| 4,14 | 5 |
| 3,80 | 24 |
| 3,74 | 100 |
| 3,56 | 65 |
| 3,31 | 22 |
| 3,15 | 45 |

TABELLE 2 (Fortsetzung)

| Netzebenenabstände d (Å) | relative Intensität 100 $I/I_o$ |
|---|---|
| 2,91 | 2 |
| 2,86 | 65 |
| 2,84 | 79 |
| 2,80 | 18 |
| 2,67 | 24 |

*Beispiel 2*

11,2 g Natriumaluminat (54 Gew.-% $Al_2O_3$, 41 Gew.-% $Na_2O$), 5,9 g Natriumhydroxid, 5,3 g Kaliumhydroxid, 48,7 g Triethanolamin und 31 g Methanol werden in 150 ml Wasser gelöst. In diese Lösung werden zuerst 100 g 40 gew.-%iges kolloidales Kieselgel und anschliessend 22,2 g Titantetrachlorid eingebracht. Die entstandene Mischung wird homogenisiert und 226 h im geschlossenen Gefäss auf 140°C erhitzt. Das entstandene Produkt wird abfiltriert, mit Wasser gewaschen und bei 120°C getrocknet.

Das Produkt zeigt die in Tabelle 1 angegebenen Röntgendaten und besitzt die folgende chemische Zusammensetzung, ausgedrückt in Molverhältnissen von Oxiden:

$SiO_2 : 0,180\ TiO_2 : 0,125\ Al_2O_3$

*Beispiel 3*

11,2 g Natriumaluminat, 5,9 g Natriumhydroxid, 5,3 g Kaliumhydroxid und 34,3 g Diethanolamin werden in 150 ml Wasser gelöst. In diese Lösung werden nacheinander 110 g 40 gew.-%iges kolloidales Kieselgel, eine Lösung aus 17,8 g Titanethanolat $Ti(OC_2H_5)_4$ in 85 g Ethanol sowie 2 g Impfkristalle aus Versuch 1 eingebracht. Die entstandene Mischung wird homogenisiert und 192 h im geschlossenen Gefäss auf 150°C erhitzt. Das entstandene Produkt wird abfiltriert, mit Wasser gewaschen und bei 120°C getrocknet. Das Produkt besitzt das in Tabelle 1 angegebene Röntgenbeugungsmuster und folgende chemische Zusammensetzung, ausgedrückt in Molverhältnissen von Oxiden:

$SiO_2 : 0,100\ TiO_2 : 0,132\ Al_2O_3$.

*Beispiel 4*

17,92 g Natriumaluminat (54 Gew.-% $Al_2O_3$, 41 Gew.-% $Na_2O$), 9,5 g Natriumhydroxid, 10 g Kaliumhydroxid, 77,6 g Triethanolamin und 96 g Ethylenglykol werden in 240 ml Wasser gelöst. In diese Lösung werden unter intensivem Rühren zuerst 178 g 40 gew.-%iges kolloidales Kieselgel und anschliessend 17,7 g Zirkonsulfat in wenig Wasser eingebracht. Die entstandene Mischung wird homogenisiert und anschliessend in einem Rührautoklaven 120 h auf 150°C erhitzt. Das entstandene Produkt wird abfiltriert, mit Wasser gewaschen und bei 120°C getrocknet.

Das Produkt besitzt das in Tabelle 1 wiedergegebene Röntgenbeugungsmuster.

Die chemische Analyse ergibt folgende Zusammensetzung, ausgedrückt in Molverhältnissen von Oxiden:

$SiO_2$ : 0,143 $Al_2O_3$ : 0,049 $ZrO_2$ : 0,052 $Na_2O$ : 0,068 $K_2O$ : 0,062 $R_2O$,

wobei R gleich $(HOCH_2CH_2)_4N$ ist.

## Patentansprüche

1. Titan-, zirkon- und/oder hafniumhaltige Zeolithe, dadurch gekennzeichnet, dass sie

a) Silicium, Aluminium, Natrium, Kalium, mindestens ein Element aus der Gruppe Titan, Zirkon und Hafnium sowie eine organische Ammoniumverbindung in folgendem Verhältnis enthalten

$(SiO_2 + MO_2)$ : (0,02 — 0,30) $Al_2O_3$ : (0,05 — 0,30) $(Na_2O + K_2O)$ : (0,01 — 0,30) $R_2O$,

ausgedrückt in Molverhältnissen von Oxiden, wobei M gleich Titan, Zirkon und/oder Hafnium ist und R Ammoniumreste der allgemeinen Formeln $(HOCH_2CH_2)_4N$, $(HOCH_2CH_2)_3R^1N$ oder $(HOCH_2CH_2)_2R^1R^2N$ bezeichnet und die Reste $R^1$ und $R^2$ gleich oder verschieden sein können und Alkyl, substituiertes Alkyl, Cycloalkyl, substituiertes Cycloalkyl, Aryl, substituiertes Aryl oder Wasserstoff bedeuten und

b) Im Röntgenbeugungsdiagramm die folgenden charakteristischen Signale aufweisen:

TABELLE 1

| Netzebenenabstände d (Å) | relative Intensität $I/I_o$ |
|---|---|
| 11,5 ± 0,3 | stark bis sehr stark |
| 9,2 ± 0,2 | schwach |
| 7,6 ± 0,2 | schwach bis mittel |
| 6,6 ± 0,1 | mittel bis stark |
| 6,3 ± 0,1 | schwach |
| 5,7 ± 0,1 | schwach |
| 5,35 ± 0,1 | schwach |
| 4,56 ± 0,1 | schwach bis mittel |
| 4,32 ± 0,1 | stark |
| 4,16 ± 0,1 | schwach |
| 3,81 ± 0,1 | mittel bis stark |
| 3,75 ± 0,1 | stark bis sehr stark |
| 3,59 ± 0,1 | stark bis sehr stark |
| 3,30 ± 0,1 | mittel |
| 3,15 ± 0,1 | mittel |
| 2,86 ± 0,1 | stark bis sehr stark |
| 2,80 ± 0,1 | schwach bis mittel |
| 2,67 ± 0,1 | schwach bis mittel |
| 2,49 ± 0,1 | schwach bis mittel |

wobei $I_o$ die Intensität des stärksten Signals bedeutet, und bei denen

c) $\frac{SiO_2}{SiO_2 + MO_2}$ = 0,4 — 0,99 gilt, ausgedrückt in Molverhältnissen der Oxide.

2. Titan-, zirkon-, und/oder hafniumhaltige Zeolithe nach Anspruch 1, dadurch gekennzeichnet, dass sie die folgende Zusammensetzung besitzen:

$(SiO_2 + MO_2)$ : (0,08 — 0,18) $Al_2O_3$ : (0,05 — 0,30) $(Na_2O + K_2O)$ : (0,01 — 0,30) $R_2O$.

3. Titan-, zirkon- und/oder hafniumhaltige Zeolithe nach einem der Ansprüche 1 oder 2, dadurch gekennzeichnet, dass $R^1$ und $R^2$ Alkylreste mit maximal jeweils fünf C-Atomen oder Wasserstoff sind.

4. Titan-, zirkon- und/oder hafniumhaltige Zeolithe nach einem der Ansprüche 1 oder 2, dadurch gekennzeichnet, dass $R^1$ und $R^2$ Methyl, Ethyl oder Wasserstoff sind.

5. Titan-, zirkon- und/oder hafniumhaltige Zeolithe nach einem der Ansprüche 1 bis 4, dadurch gekennzeichnet, dass $R^1$ gleich $R^2$ ist.

6. Titan-, zirkon- und/oder hafniumhaltige Zeolithe nach einem der Ansprüche 1 oder 2, dadurch gekennzeichnet, dass $R^1$ und $R^2$ gleich Methyl sind.

7. Titan-, zirkon- und/oder hafniumhaltige Zeolithe nach einem der Ansprüche 1 oder 2, dadurch gekennzeichnet, dass gilt:

$$\frac{SiO_2}{SiO_2 + MO_2} = 0{,}7 — 0{,}99,$$

augedrückt in Molverhältnissen der Oxide.

8. Verfahren zur Herstellung von titan-, zirkon- und/oder hafniumhaltigen Zeolithen nach einem der Ansprüche 1 bis 7, dadurch gekennzeichnet, dass man eine Mischung aus Silicium-, Aluminium-, Natrium-, Kalium-, organischen Ammoniumverbindungen und Wasser sowie mindestens einer Verbindung aus der Gruppe der Titan-, Zirkon- und/oder Hafniumverbindungen herstellt, die folgende Zusammensetzung hat, ausgedrückt in Molverhältnissen der Oxide:

$(SiO_2 + MO_2)$ : (0,02 — 0,30) $Al_2O_3$ : (0,02 — 0,70) $Na_2O$ : (0,02 — 0,30) $K_2O$ : (0,02 — 0,50) $R_2O$ : (10 — 90) $H_2O$,

und wobei für das Gemisch der Ausgangsverbindungen gilt:

$$\frac{SiO_2}{SiO_2 + MO_2} = 0{,}4 — 0{,}99,$$

ausgedrückt in Molverhältnissen der Oxide und diese Mischung in einem geschlossenen Gefäss erhitzt.

9. Verfahren nach Anspruch 8, dadurch gekennzeichnet, dass die zu erhitzende Mischung folgende Zusammensetzung hat, ausgedrückt in Molverhältnissen der Oxide:

$(SiO_2 + MO_2)$ : (0,02 — 0,18) $Al_2O_3$ : (0,10 — 0,60) $Na_2O$ : (0,04 — 0,20) $K_2O$ : (0,10 — 0,40) $R_2O$ : (10 — 40) $H_2O$.

10. Verfahren nach einem der Ansprüche 8 oder 9, dadurch gekennzeichnet, dass man anstelle einer Ammoniumverbindung eine äquivalente Menge an Triethanolamin und/oder Diethanolamin zusammen mit einer Verbindung der allgemeinen Formel $R^1Y$ einsetzt, wobei $R^1$ gleich Alkyl, substituiertes Alkyl, Cycloalkyl, substituiertes Cycloalkyl, Aryl, substituiertes Aryl oder Wasserstoff ist und Y gleich Hydroxyl, Monoalkylsulfat, Halogenid oder Sulfonat ist.

11. Verfahren nach Anspruch 10, dadurch gekennzeichnet, dass $R^1Y$ gleich Methanol, Ethanol,

Propanol, Butanol, Ethylenglykol, 1,2-Propylenglykol, Dimethylsulfat, Diethylsulfat, Methyliodid, Ethyliodid, Propyliodid, p-Toluolsulfonsäuremethylester, p-Toluolsulfonsäureethylester oder p-Toluolsulfonsäurepropylester ist.

12. Verfahren nach Anspruch 10, dadurch gekennzeichnet, dass $R^1Y$ gleich Methanol, Ethanol oder Ethylenglykol ist.

13. Verfahren nach einem der Ansprüche 8 oder 9, dadurch gekennzeichnet, dass man anstelle einer Ammoniumverbindung eine äquivalente Menge an Triethanolamin einsetzt.

14. Verfahren nach einem der Ansprüche 8 oder 9, dadurch gekennzeichnet, dass man anstelle einer Ammoniumverbindung eine äquivalente Menge an Diethanolamin einsetzt.

15. Verfahren nach einem der Ansprüche 8 oder 9, dadurch gekennzeichnet, dass für das Gemisch der Ausgangsverbindungen gilt:

$$\frac{SiO_2}{SiO_2 + MO_2} = 0{,}6 - 0{,}99,$$

ausgedrückt in Molverhältnissen der Oxide.

16. Verwendung von titan-, zirkon- und/oder hafniumhaltigen Zeolithen nach einem der Ansprüche 1 bis 7 als Katalysatoren bei der Herstellung von $C_2$- bis $C_4$-Olefinen aus Methanol.

## Claims

1. A titanium-, zirconium- and/or hafnium-containing zeolite which

a) contains silicon, aluminum, sodium, potassium, at least one element from the group consisting of titanium, zirconium and hafnium and an organic ammonium compound in the following ratio:

$(SiO_2 + MO_2) : (0.02 - 0.30)\ Al_2O_3 : (0.05 - 0.30)\ (Na_2O + K_2O) : 0.01 - 0.30)\ R_2O$,

expressed in molar ratios of the oxides, where M is equal to titanium, zirconium and/or hafnium, R denotes ammonium radicals of the general formulae $(HOCH_2CH_2)_4N$, $(HOCH_2CH_2)_3R^1N$ or $(HOCH_2CH_2)_2R^1R^2N$, and the radicals $R^1$ and $R^2$ can be identical or different and denote alkyl, substituted alkyl, cycloalkyl, substituted cycloalkyl, aryl, substituted aryl or hydrogen, and

b) has, in the X-ray diffraction diagram, the characteristic signals listed in Table 1:

TABLE 1

| Lattice plane distances d (Å) | Relative intensity $I/I_o$ |
|---|---|
| 11.5 ± 0.3 | strong to very strong |
| 9.2 ± 0.2 | weak |
| 7.6 ± 0.2 | weak to medium |
| 6.6 ± 0.1 | medium to strong |
| 6.3 ± 0.1 | weak |
| 5.7 ± 0.1 | weak |
| 5.35 ± 0.1 | weak |
| 4.56 ± 0.1 | weak to medium |
| 4.32 ± 0.1 | strong |
| 4.16 ± 0.1 | weak |
| 3.81 ± 0.1 | medium to strong |
| 3.75 ± 0.1 | strong to very strong |
| 3.59 ± 0.1 | strong to very strong |
| 3.30 ± 0.1 | medium |
| 3.15 ± 0.1 | medium |
| 2.86 ± 0.1 | strong to very strong |
| 2.80 ± 0.1 | weak to medium |
| 2.67 ± 0.1 | weak to medium |
| 2.49 ± 0.1 | weak to medium |

where in the Table, $I_o$ denotes the intensity of the strongest signal and wherein

c) $$\frac{SiO_2}{SiO_2 + MO_2} = 0.4 - 0.99,$$

expressed in molar ratios of the oxides.

2. A titanium-, zirkonium- and/or hafnium-containing zeolite as claimed in claim 1, which has the following composition:

$(SiO_2 + MO_2) : (0.08 - 0.18)\ Al_2O_3 : (0.05 - 0.30)\ (Na_2O + K_2O) : (0.01 - 0.30)\ R_2O$.

3. A titanium-, zirconium- and/or hafnium-containing zeolite as claimed in either of claims 1 or 2, wherein $R^1$ and $R^2$ are alkyl radicals of at most 5 carbon atoms each or hydrogen.

4. A titanium-, zirconium- and/or hafnium-containing zeolite as claimed in either of claims 1 or 2, wherein $R^1$ and $R^2$ are methyl, ethyl or hydrogen.

5. A titanium-, zirconium- and/or hafnium-containing zeolite as claimed in any one of claims 1 to 4, wherein $R^1$ is equal to $R^2$.

6. A titanium-, zirconium- and/or hafnium-containing zeolite as claimed in either of claims 1 or 2, wherein $R^1$ and $R^2$ are equal to methyl.

7. A titanium-, zirconium- and/or hafnium-containing zeolite as claimed in either of claims 1 or 2, wherein

$$\frac{SiO_2}{SiO_2 + MO_2} = 0.7 - 0.99,$$

expressed in molar ratios of the oxides.

8. A process for preparing a titanium-, zirconium- and/or hafnium-containing zeolite as claimed in any one of claims 1 to 7, which comprises preparing a mixture of silicon, aluminum, sodium, potassium, and organic ammonium compounds and water as well as at least one compound from the group consisting of titanium, zirconium and/or hafnium compounds, which has the following composition as expressed in molar ratios of the oxides:

$(SiO_2 + MO_2) : (0.02 - 0.30)\ Al_2O_3 : (0.02 - 0.70)\ Na_2O : (0.02 - 0.30)\ K_2O : (0.02 - 0.50)\ R_2O : (10 - 90)\ H_2O$,

and wherein the mixture of the starting compounds is in accordance with:

$$\frac{SiO_2}{SiO_2 + MO_2} = 0.4 — 0.99,$$

expressed in molar ratios of the oxides, and heating this mixture in a sealed vessel.

9. The process as claimed in claim 8, wherein the mixture to be heated has the following composition as expressed in molar ratios of the oxides:

$(SiO_2 + MO_2 : (0.02 — 0.18)\ Al_2O_3 : (0.10 — 0.60)\ Na_2O : (0.04 — 0.20)\ K_2O : (0.10 — 0.40)\ R_2O : (10 — 40)\ H_2O$.

10. The process as claimed in either of claims 8 or 9, wherein the ammonium compound is replaced by an equivalent amount of triethanolamine and/or diethanolamine together with a compound of the formula $R^1Y$ where $R^1$ is equal to alkyl, substituted alkyl, cycloalkyl, substituted cycloalkyl, aryl, substituted aryl or hydrogen, and Y is equal to hydroxyl, monoalkyl sulfate, halide or sulfonate.

11. The process as claimed in claim 10, wherein $R^1Y$ is equal to methanol, ethanol, propanol, butanol, ethylene glycol, 1,2-propylene glycol, dimethyl sulfate, diethyl sulfate, methyl iodide, ethyl iodide, propyl iodide, methyl p-toluenesulfonate, ethyl p-toluenesulfonate or propyl p-toluene sulfonate.

12. The process as claimed in claim 10, wherein $R^1Y$ is equal to methanol, ethanol or ethylene glycol.

13. The process as claimed in either of claims 8 or 9, wherein the ammonium compound is replaced by an equivalent amount of triethanolamine.

14. The process as claimed in either of claims 8 or 9, wherein the ammonium compound is replaced by an equivalent amount of diethanolamine.

15. The process as claimed in either of claims 8 or 9, wherein the mixture of the starting compounds is in accordance with:

$$\frac{SiO_2}{SiO_2 + MO_2} = 0.6 — 0.99,$$

expressed in molar ratios of the oxides.

16. Use of a titanium-, zirconium- and/or hafnium-containing zeolite as claimed in any one of claims 1 to 7, as a catalyst in the preparation of $C_2$- to $C_4$-olefins from methanol.

**Revendications**

1. Zéolites contenant du titane, du zirconium et/ou du hafnium, caractérisées en ce qu'elles:

a) contiennent du silicium, de l'aluminium, du sodium, du potassium, au moins un élément de l'ensemble formé par le titane, le zirconium et le hafnium, ainsi qu'un composé organique d'ammonium selon le rapport suivant:

$(SiO_2 + MO_2) : (0,02 — 0,30)\ Al_2O_3 : (0,05 — 0,30)\ (Na_2O + K_2O) : (0,01 — 0,30)\ R_2O$,

exprimé en les rapports molaires des oxydes, où M représente le titane, le zirconium et/ou le hafnium et R représente des restes ammonium répondant aux formules générales $(HOCH_2CH_2)_4N$, $(HOCH_2CH_2)_3R^1N$ ou $(HOCH_2CH_2)_2R^1R^2N$, et les restes $R^1$ et $R^2$ peuvent être identiques ou différents et désignent des groupes alkyles, alkyles substitués, cycloalkyles, cycloalkyles substitués, aryles, aryles substitués ou des atomes d'hydrogène, et

b) pésentent dans un diagramme de diffraction des rayons X les signaux caractéristiques suivants:

TABLEAU I

| Distances entre plans réticulaires d (Å) | | Intensité relative $I/I_o$ |
|---|---|---|
| 11,5 | ± 0,3 | forte à très forte |
| 9,2 | ± 0,2 | faible |
| 7,6 | ± 0,2 | faible à moyenne |
| 6,6 | ± 0,1 | moyenne à forte |
| 6,3 | ± 0,1 | faible |
| 5,7 | ± 0,1 | faible |
| 5,35 | ± 0,1 | faible |
| 4,56 | ± 0,1 | faible à moyenne |
| 4,32 | ± 0,1 | forte |
| 4,16 | ± 0,1 | faible |
| 3,81 | ± 0,1 | moyenne à forte |
| 3,75 | ± 0,1 | forte à très forte |
| 3,59 | ± 0,1 | forte à très forte |
| 3,30 | ± 0,1 | moyenne |
| 3,15 | ± 0,1 | moyenne |
| 2,86 | ± 0,1 | forte à très forte |
| 2,80 | ± 0,1 | faible à moyenne |
| 3,67 | ± 0,1 | faible à moyenne |
| 2,49 | ± 0,1 | faible à moyenne |

$I_o$ représentant l'intensité du signal le plus intense, et

c) elles présentent une valeur du rapport

$$\frac{SiO_2}{SiO_2 + MO_2} \text{ égale à 0,4 à 0,99,}$$

quand se rapport est exprimé en les rapports molaires des oxydes.

2. Zéolites contenant du titane, du zirconium et/ou du hafnium selon la revendication 1, caractérisées en ce qu'elles possèdent la composition suivante:

$(SiO_2 + MO_2) : (0,08 — 0,18)\ Al_2O_3 : (0,05 — 0,30)\ (Na_2O + K_2O) : (0,01 — 0,30)\ R_2O$.

3. Zéolites contenant du titane, du zirconium et/ou de l'hafnium selon l'une des revendications 1 ou 2, caractérisées en ce que $R^1$ et $R^2$ représentent des restes alkyles ayant chacun au maximum cinq atomes de carbone ou un atome d'hydrogène.

4. Zéolites contenant du titane, du zirconium et/ou de l'hafnium selon l'une des revendications 1 ou 2, caractérisées en ce que $R^1$ et $R^2$ représentent un groupe méthyle, éthyle ou un atome d'hydrogène.

5. Zéolites contenant du titane, du zirconium

et/ou de l'hafnium selon l'une des revendications 1 à 4, caractérisées en ce que $R^1$ est identique à $R^2$.

6. Zéolites contenant du titane, du zirconium et/ou de l'hafnium selon l'une des revendications 1 ou 2, caractérisées en ce que $R^1$ et $R^2$ représentent chacun un groupe méthyle.

7. Zéolites contenant du titane, du zirconium et/ou de l'hafnium selon l'une des revendications 1 ou 2, caractérisées en ce que le rapport:

$$\frac{SiO_2}{SiO_2 + MO_2}$$

exprimé en les rapports molaires des oxydes, vaut de 0,7 à 0,99.

8. Procédé pour préparer des zéolites contenant du titane, du zirconium et/ou de l'hafnium selon l'une des revendications 1 à 7, caractérisé en ce qu'on prépare un mélange de composés de silicium, d'aluminium, de sodium, de potassium, de composés organiques d'ammonium et de l'eau ainsi qu'au moins un composé choisi parmi les composés de titane, du zirconium et/ou de l'hafnium, ayant la composition suivante, exprimée en rapport molaire des oxydes:

$(SiO_2 + MO_2) : (0{,}02 — 0{,}30)\ Al_2O_3 : (0{,}02 — 0{,}70)\ Na_2O : (0{,}02 — 0{,}30)\ K_2O : (0{,}02 — 0{,}50)\ R_2O : (10 — 90)\ H_2O$,

et, pour le mélange des composées de départ, le rapport

$$\frac{SiO_2}{SiO_2 + MO_2}$$

exprimé en les rapports molaires des oxydes, vaut de 0,4 à 0,99, et l'on chauffe ce mélange dans un récipient fermé.

9. Procédé selon la revendication 8, caractérisé en ce que le mélange à chauffer possède la composition suivante, exprimée en rapports molaires des oxydes:

$(SiO_2 + MO_2) : (0{,}02 — 0{,}18)\ Al_2O_3 : (0{,}10 — 0{,}60)\ Na_2O : (0{,}04 — 0{,}20)\ K_2O : (0{,}10 — 0{,}40)\ R_2O : (10 — 40)\ H_2O$.

10. Procédé selon l'une des revendications 8 ou 9, caractérisé en ce qu'au lieu d'un composé d'ammonium, on utilise une quantité équivalente de triéthanolamine et/ou de diéthanolamine avec un composé de formule générale $R^1Y$, dans laquelle $R^1$ représente un groupe alkyle, alkyle substitué, cycloalkyle, cycloalkyle substitué, aryle, aryle substitué ou un atome d'hydrogène, et Y représente un groupe hydroxyle, monoalkyl sulfate, halogénure ou sulfonate.

11. Procédé selon la revendication 10, caractérisé en ce que $R^1Y$ représente le méthanol, l'éthanol, le propanol, le butanol, l'éthylène-glycol, le propylène-glycol-1,2, le sulfate de diméthyle, le sulfate de diéthyle, l'iodure de méthyle, l'iodure d'éthyle, l'iodure de propyle, le p-toluènesulfonate de méthyle, le p-toluènesulfonate d'éthyle ou le p-toluènesulfonate de propyle.

12. Procédé selon la revendication 10, caractérisé en ce que $R^1Y$ représente le méthanol, l'éthanol ou l'éthylèneglycol.

13. Procédé selon l'une des revendications 8 ou 9, caractérisé en ce qu'au lieu d'un composé d'ammonium, on utilise une quantité équivalente de triéthanolamine.

14. Procédé selon l'une des revendications 8 ou 9, caractérisé en ce qu'au lieu d'un composé d'ammonium, on utilise une quantité équivalente de diéthanolamine.

15. Procédé selon l'une des revendications 8 ou 9, caractérisé en ce que, pour le mélange des composés de départ, le rapport:

$$\frac{SiO_2}{SiO_2 + MO_2}$$

exprimé en les rapports molaires des oxydes, vaut de 0,6 à 0,99.

16. Utilisation de zéolites contenant du titane, du zirconium et/ou de l'hafnium selon l'une des revendications 1 à 7, comme catalyseurs dans la production d'oléfines en $C_2$ à $C_4$ à partir du méthanol.